# EUROPEAN PATENT APPLICATION

(11) **EP 4 813 404 A1**
(43) Date of publication of application: **30.09.2026**
(21) Application number: 24894132.0
(22) Date of filing: 19.11.2024
(51) Int. Cl.: A61K 38/17, A23K 20/147, A23L 33/18, A61P 25/20, C07K 14/47

(54) **SLEEP-IMPROVING AGENT**

(30) Priority: 21.11.2023 JP 2023197697
(71) Applicant: National University Corporation University of Toyama, Toyama-shi, Toyama 930-8555 (JP)
(72) Inventor: TSUNEKI, Hiroshi, Toyama-shi, Toyama 930-0194 (JP); SASAOKA, Toshiyasu, Toyama-shi, Toyama 930-0194 (JP); ITO, Hisakatsu, Toyama-shi, Toyama 930-0194 (JP)
(74) Representative: Müller-Boré & Partner Patentanwälte PartG mbB
(86) International application number: PCT/JP2024/040910
(87) International publication number: WO 2025/110141

(57) **Abstract**

An agent for sleep improvement contains any one of the following peptides (I) to (IV):
(I) a peptide containing the amino acid sequence of the C-terminal peptide of preproorexin;
(II) a peptide having at least 90% amino acid sequence identity to the amino acid sequence of the C-terminal peptide of preproorexin of the peptide (I);
(III) a peptide that is a part of the amino acid sequence of the C-terminal peptide of preproorexin of the peptide (I); and
(IV) a variant of the peptide (I) or (III) in which one or two amino acids are deleted, substituted, added, or inserted with respect to the peptide (I) or (III).

## Description

### Technical Field

The present invention relates to an agent for sleep improvement.

### Background Art

The sleep-wake cycle is the foundation of biological rhythms, and its disruption is a triggering factor not only for sleep disorders but also for various diseases such as depression and diabetes. The hypothalamic orexin neural system secretes the neuropeptides orexin-A and orexin-B, and plays an essential role in maintaining wakefulness through activation of orexin 1 receptor and orexin 2 receptor in the brain. Orexin deficiency thus causes hypersomnia (narcolepsy). Accordingly, drugs that enhance the wakefulness-maintaining action of orexin may be effective in the treatment of hypersomnia. On the other hand, antagonists that inhibit both orexin 1 receptor and orexin 2 receptor (e.g., suvorexant and lemborexant) suppress wakefulness and induce non-REM sleep and REM sleep, and are therefore clinically used as therapeutic agents for insomnia. Drugs that inhibit orexin action without involving orexin receptors (e.g., physiological antagonists) may also be effective for the treatment of insomnia.

Preproorexin expressed by hypothalamic neurons in the brain is fragmented by post-translational modification to generate orexin-A, orexin-B, and a peptide composed of 31 amino acids in the C-terminal region of preproorexin (orexin-C). Orexin-A and orexin-B were identified as physiologically active substances in evaluation systems using HEK293 cells or CHO cells in which orexin receptors were forcibly expressed (NPL 1). The literature stated that orexin-C was not considered a physiologically active peptide, even though its activity had not been thoroughly evaluated. The present inventors previously found that the C-terminal peptide of preproorexin (orexin-C) increases brain-derived neurotrophic factors in cerebral cortex neurons and suppresses feeding in mice through central nervous system action, and they discovered that it is a peptide having activity (PTL 1).

However, there are no reports that have examined the involvement of orexin-C in sleep and wakefulness.

### Citation List

### Patent Literature

PTL 1: JP2023-135605A

### Non-patent Literature

NPL 1: Cell 1998; 92: 573-585

### Summary of Invention

### Technical Problem

An object to be achieved by the present invention is to provide an agent for sleep improvement containing a substance having an effect of improving sleep as an active ingredient.

### Solution to Problem

The present invention encompasses the embodiments described below.
Item 1. An agent for sleep improvement, comprising any one of the following peptides (I) to (IV),
   wherein
   the peptide (I) is a peptide containing the amino acid sequence of the C-terminal peptide of preproorexin,
   the peptide (II) is a peptide having at least 90% amino acid sequence identity to the amino acid sequence of the C-terminal peptide of preproorexin of the peptide (I),
   the peptide (III) is a peptide that is a part of the amino acid sequence of the C-terminal peptide of preproorexin of the peptide (I), and
   the peptide (IV) is a variant of the peptide (I) or (III) in which one or two amino acids are deleted, substituted, added, or inserted with respect to the peptide (I) or (III). Item 2.
   The agent for sleep improvement according to Item 1, wherein the peptide (I) is a peptide containing the amino acid sequence of any one of SEQ ID NOs: 1 to 3.
Item 3. The agent for sleep improvement according to Item 1, which is a sleep inducer.
Item 4. The agent for sleep improvement according to Item 1, wherein the sleep improvement includes an improvement in sleep depth or an improvement in asleep onset.
Item 5. The agent for sleep improvement according to Item 1, which is an agent for the prevention or treatment of a sleep disorder.
Item 6. The agent for sleep improvement according to any one of Items 1 to 5, which is in the form of a pharmaceutical composition.
Item 7. The agent for sleep improvement according to any one of Items 1 to 5, which is in the form of a food composition.
Item 8. The agent for sleep improvement according to any one of Items 1 to 5, which is in the form of feed.
Item 9. A method for improving sleep, comprising administering to a subject any one of the following peptides (I) to (IV), excluding a method for treating a human:
   (I) a peptide containing the amino acid sequence of the C-terminal peptide of preproorexin,
   (II) a peptide having at least 90% amino acid sequence identity to the amino acid sequence of the C-terminal peptide of preproorexin of the peptide (I),
   (III) a peptide that is a part of the amino acid sequence of the C-terminal peptide of preproorexin of the peptide (I), and
   (IV) a variant of the peptide (I) or (III) in which one or two amino acids are deleted, substituted, added, or inserted with respect to the peptide (I) or (III).
Item 10. The method according to Item 9, wherein the sleep improvement includes prevention or treatment of a sleep disorder.

### Advantageous Effects of Invention

The agent for sleep improvement of the present invention improves a sleep disorder due to the inclusion of orexin-C, a variant peptide thereof, or a partial peptide thereof as an active ingredient.

### Brief Description of Drawings

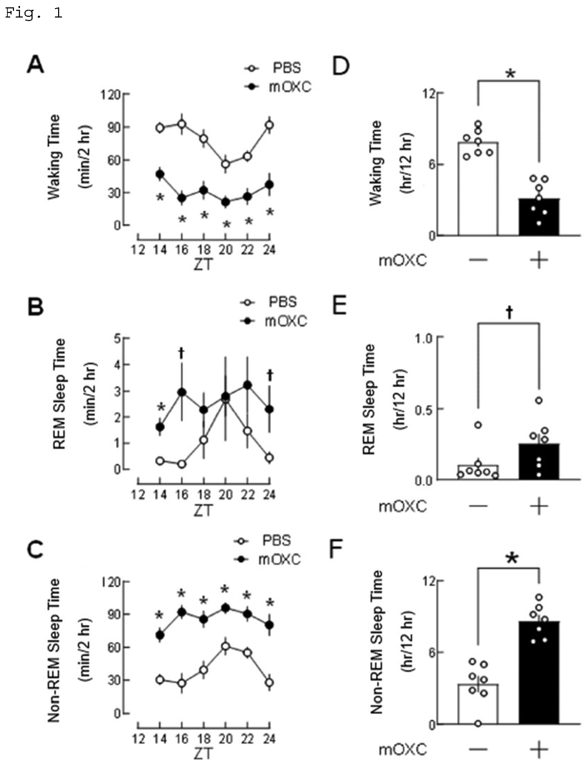

Fig. 1 shows a sleep-inducing effect of orexin-C in mice. Fig. 1 indicates wakefulness time (A, D), REM sleep time (B, E), and non-REM sleep time (C, F) after intracerebroventricular administration of mouse orexin-C (mOXC) (closed circles) or phosphate-buffered saline (PBS) as a control (open circles) at ZT12 to C57BL/6J mice. (A-C) Wakefulness time, REM sleep time, and non-REM sleep time were measured as cumulative values every 2 hours. (D) Total of wakefulness time during 12 hours in (A). (E) Total of REM sleep time during 12 hours in (B). (F) Total of non-REM sleep time during 12 hours in (C). Total n = 7/group. *P < 0.05, †P < 0.1: A significant difference was detected using Student's paired *t*-test.

### Description of Embodiments

In the present specification, the term "comprising" is a concept that also encompasses consisting essentially of and consisting of.

In the present specification, the term "administration" to a subject includes ingestion by a subject.

In the present specification, the term "peptide" refers to a molecule in which 2 or more and less than 50 amino acids are bonded. A molecule in which 10 or more amino acids are bonded is particularly referred to as a "polypeptide."

In the present specification, a "protein" refers to a molecule in which 50 or more amino acids are bonded.

In the present specification, the phrase "the C-terminal peptide of preproorexin" refers to a peptide composed of an amino acid sequence including orexin-A, a GKR sequence, orexin-B, and a region from the next amino acid following the GKR sequence or GRR sequence to the last amino acid, among the amino acid sequence of preproorexin. The C-terminal peptide of preproorexin is also referred to as "orexin gene-related peptide" (OGRP) or "orexin-C" (OXC).

An embodiment for performing the present invention is described below. The embodiment described below is an example of a typical embodiment of the invention, and the scope of the present invention should not be narrowly construed thereby.

Preproorexin expressed by hypothalamic neurons in the brain is fragmented by post-translational modification to generate orexin-A, orexin-B, and a C-terminal peptide composed of 31 amino acids in the C-terminal region of preproorexin (orexin-C). The research conducted by the present inventors disclosed in JP2023-135605A left open the possibility that orexin-C exhibits physiological activity through biomolecules other than orexin 1 receptor or orexin 2 receptor. The inventors focused on the fact that orexin-A and orexin-B are factors that maintain wakefulness, and revealed that orexin-C is also a factor that affects sleep and/or wakefulness.

The amino acid sequence of preproorexin corresponds to UniProt accession No. O55232 in rats, UniProt accession No. O55241 in mice, and UniProt accession No. O43612 in humans. The amino acid sequences of orexin-C of rats, mice, and humans are as follows:
Rat orexin-C (rOXC): AGAELEPYPCPGRRCPTATATALAPRGGSRV (SEQ ID NO: 1)
Mouse orexin-C (mOXC): AGAELEPHPCSGRGCPTVTTTALAPRGGSGV (SEQ ID NO: 2)
Human orexin-C (hOXC): AGAEPAPRPCLGRRCSAPAAASVAPGGQSGI (SEQ ID NO: 3)

In one aspect of the present invention, there is provided an agent for sleep improvement containing any one of the following peptides (I) to (IV):
(I) a peptide containing the amino acid sequence of the C-terminal peptide of preproorexin,
(II) a peptide having at least 90% amino acid sequence identity to the amino acid sequence of the C-terminal peptide of preproorexin of the peptide (I),
(III) a peptide that is a part of the amino acid sequence of the C-terminal peptide of preproorexin of the peptide (I), and
(IV) a variant of the peptide (I) or (III) in which one or two amino acids are deleted, substituted, added, or inserted with respect to the peptide (I) or (III).

The agent for sleep improvement of the present invention contains any one of the peptides (I) to (IV) as an active ingredient. Either of the peptides (I) to (IV) is useful for improving sleep. Sleep improvement includes an improvement in sleep quality, sleep quantity, or both. Further, sleep improvement includes, for example, any one of the following: induction of sleep, promotion of sleep initiation, suppression of awakening during sleep, an improvement in sleep depth, and an increase in sleep duration. The increase in sleep duration includes an increase in REM sleep time, an increase in non-REM sleep time, and an increase in both, and preferably includes an increase in non-REM sleep time.

The agent for sleep improvement in this aspect of the present invention may contain one peptide among the peptides (I) to (IV), or may contain two or more peptides among them. When the agent for sleep improvement in this aspect of the present invention contains two or more peptides among the peptides (I) to (IV), the amount of peptides refers to the total amount of the peptides (I) to (IV).

In some embodiments, the peptide (I) is a polypeptide composed only of the amino acid sequence of the C-terminal peptide of preproorexin.

In some embodiments, the peptide (I) is a polypeptide containing the amino acid sequence of any one of SEQ ID NOs: 1 to 3.

In some embodiments, the peptide (I) is a polypeptide composed only of the amino acid sequence of any one of SEQ ID NOs: 1 to 3.

In some embodiments, the peptide (II) is a polypeptide having at least 90% amino acid sequence identity to the amino acid sequence of the C-terminal peptide of preproorexin of the peptide (I) and having a sleep-improving effect. The sleep-improving effect includes, for example, an improvement in sleep in a subject compared with a case in which the polypeptide is not administered to the subject.

In some embodiments, the peptide (III) is a peptide that is a contiguous portion of the amino acid sequence of the C-terminal peptide of preproorexin of the peptide (I), and is preferably a polypeptide.

In some embodiments, the peptide (III) is a peptide that is a contiguous portion of the amino acid sequence of the C-terminal peptide of preproorexin of the peptide (I) and is composed of 5 to 30 amino acids; this peptide (III) is preferably a polypeptide.

In some embodiments, the peptide (III) is a peptide that is a contiguous portion of the amino acid sequence of the C-terminal peptide of preproorexin of the peptide (I) and is composed of 8 to 30 amino acids; this peptide (III) is preferably a polypeptide.

In some embodiments, the peptide (III) is a peptide that is a contiguous portion of the amino acid sequence of the C-terminal peptide of preproorexin of the peptide (I) and has a sleep-improving effect; this peptide (III) is preferably a polypeptide. The sleep-improving effect includes, for example, an improvement in sleep in a subject compared with a case in which the polypeptide is not administered to the subject.

In some embodiments, the peptide (IV) is a variant of the peptide (I) or (III) in which one or two amino acids are deleted, substituted, added, or inserted with respect to the peptide (I) or (III), and has a sleep-improving effect; this peptide (IV) is preferably a polypeptide. The sleep-improving effect includes, for example, an improvement in sleep in a subject compared with a case in which the peptide is not administered to the subject.

In some embodiments, the peptide (IV) is a variant of the peptide (I) or (III) in which one or two amino acids are deleted, substituted, added, or inserted with respect to the C-terminal peptide of preproorexin of the peptide (I), and has a sleep-improving effect; this peptide (IV) is preferably a polypeptide. The sleep-improving effect includes, for example, an improvement in sleep in a subject compared with a case in which the peptide is not administered to the subject.

In some embodiments, the agent for sleep improvement in the above aspect is a sleep inducer.

In some embodiments, the sleep improvement brought by the agent for sleep improvement in the above aspect includes an improvement in sleep depth and an improvement in asleep onset.

In some embodiments, the agent for sleep improvement is an agent for the prevention or treatment of a sleep disorder.

In some embodiments, the agent for sleep improvement in the above aspect is in the form of a pharmaceutical composition. The pharmaceutical composition may contain a pharmaceutically acceptable carrier. The pharmaceutically acceptable carrier for use may be various organic or inorganic carrier substances that are commonly used as formulation materials. Examples include excipients, diluents, additives, disintegrants, binders, coating agents, lubricants, glidants, slipping agents, flavoring agents, sweeteners, and solubilizers. The pharmaceutical composition may also optionally contain formulation additives, such as preservatives, oxidation inhibitors (antioxidants), coloring agents, taste-masking agents, odor-masking agents, stabilizers, and aromatic agents.

The pharmaceutical composition may be of any dosage form that can be used in pharmaceutical formulations; for example, the pharmaceutical composition is administered orally or parenterally (e.g., intracerebrally, intravenously, or intraperitoneally). Examples of dosage forms include tablets, granules, powders, and injectable preparations.

When the agent for sleep improvement is in the form of a pharmaceutical composition, the dosage of any one of the polypeptides (I) to (IV) to be administered to a subject is preferably 0.0001 to 5000 milligrams per kilogram of subject body weight per day (mg/kg/day), more preferably 0.001 to 1000 mg/kg/day.

The daily dose may be administered orally or parenterally in a single dose or in divided doses of 2 to 4 times at appropriate intervals.

In some embodiments, the agent for sleep improvement in the above aspect is in the form of a food composition.

Foods are usually included in the food composition and refer to all foods including beverages, and include not only general foods including "health food products," but also foods for specified health uses and foods with nutrient function claims specified in the Foods with Health Claims under the Ministry of Health, Labour and Welfare. Foods also include supplements.

The food composition may contain food-acceptable carriers, such as various food processing ingredients, seasonings, flavorings, and sweeteners. The food composition may be formulated as powders, tablets, capsules, drinks, or the like. The food-acceptable carriers include the pharmaceutically acceptable carriers described above.

When the agent for sleep improvement is in the form of a food composition, the dosage of any one of the peptides (I) to (IV) to be administered to a subject is preferably 0.0001 to 5000 milligrams per kilogram of subject body weight per day (mg/kg/day), more preferably 0.001 to 1000 mg/kg/day.

The daily dose may be administered orally in a single dose or in divided doses of 2 to 4 times at appropriate intervals.

In some embodiments, the food composition contains any one of the peptides (I) to (IV) in an amount of preferably 0.0001 to 10 wt%, more preferably 0.001 to 5 wt%, and more preferably 0.01 to 1 wt%.

In some embodiments, the agent for sleep improvement in the above aspect is in the form of feed. The subject to receive the feed is a non-human animal.

When the agent for sleep improvement is in the form of feed, the dose of any one of the peptides (I) to (IV) to a subject is preferably 0.0001 to 5000 milligrams per kilogram of subject body weight per day (mg/kg/day), more preferably 0.001 to 1000 mg/kg/day.

The daily dose may be administered orally in a single dose or in divided doses of 2 to 4 times at appropriate intervals.

In another aspect of the present invention, there is provided a method for producing the agent for sleep improvement in the above aspect, and the method comprises adding any one of the peptides (I) to (IV). For example, the pharmaceutical composition, the food composition, or the feed can be produced by adding any one of the peptides (I) to (IV) described above.

In another aspect of the present invention, there is provided a method for improving sleep, comprising administering to a subject any one of the following peptides (I) to (IV): the peptide (I) is a peptide containing the amino acid sequence of the C-terminal peptide of preproorexin,
the peptide (II) is a peptide having at least 90% amino acid sequence identity to the amino acid sequence of the C-terminal peptide of preproorexin of the peptide (I),
the peptide (III) is a peptide that is a part of the amino acid sequence of the C-terminal peptide of preproorexin of the peptide (I), and
the peptide (IV) is a variant of the peptide (I) or (III) in which one or two amino acids are deleted, substituted, added, or inserted with respect to the peptide (I) or (III).

The subject may be a human or a non-human animal. The non-human animal is preferably a non-human mammal. The non-human mammal includes, but is not limited to, mice and rats. In some embodiments, the subject is a subject in need of sleep improvement.

In some embodiments, the peptide (I) is a polypeptide composed only of the amino acid sequence of the C-terminal peptide of preproorexin.

In some embodiments, the peptide (I) is a polypeptide containing the amino acid sequence of any of SEQ ID NOs: 1 to 3.

In some embodiments, the peptide (I) is a polypeptide composed only of the amino acid sequence of any of SEQ ID NOs: 1 to 3.

In some embodiments, the peptide (II) is a polypeptide having at least 90% amino acid sequence identity to the amino acid sequence of the C-terminal peptide of preproorexin of the peptide (I) and is a peptide having a sleep-improving effect. This peptide (II) is preferably a polypeptide having a sleep-improving effect. The sleep-improving effect includes, for example, an improvement in sleep in a subject compared with a case in which the peptide is not administered to the subject.

In some embodiments, the peptide (III) is a peptide that is a contiguous portion of the amino acid sequence of the C-terminal peptide of preproorexin of the peptide (I), and is preferably a polypeptide.

In some embodiments, the peptide (III) is a peptide that is a contiguous portion of the amino acid sequence of the C-terminal peptide of preproorexin of the peptide (I) and is composed of 5 to 30 amino acids, and is more preferably a polypeptide.

In some embodiments, the peptide (III) is a peptide that is a contiguous portion of the amino acid sequence of the C-terminal peptide of preproorexin of the peptide (I) and is composed of 8 to 30 amino acids, and is more preferably a polypeptide.

In some embodiments, the peptide (III) is a peptide that is a contiguous portion of the amino acid sequence of the C-terminal peptide of preproorexin of the peptide (I) and has a sleep-improving effect; this peptide (III) is preferably a polypeptide. The sleep-improving effect includes, for example, an improvement in sleep in a subject compared with a case in which the peptide is not administered to the subject.

In some embodiments, the peptide (IV) is a variant of the peptide (I) or (III) in which one or two amino acids are deleted, substituted, added, or inserted with respect to the peptide (I) or (III), and has a sleep-improving effect; this peptide (IV) is preferably a polypeptide. The sleep-improving effect includes, for example, an improvement in sleep in a subject compared with a case in which the peptide is not administered to the subject.

In some embodiments, the peptide (IV) is a variant of the peptide (I) or (III) in which one or two amino acids are deleted, substituted, added, or inserted with respect to the C-terminal peptide of preproorexin of the peptide (I), and has a sleep-improving effect; this peptide (IV) is preferably a polypeptide. The sleep-improving effect includes, for example, an improvement in sleep in a subject compared with a case in which the peptide is not administered to the subject.

Any one of the peptides (I) to (IV) can be administered in the form of the agent for sleep improvement, the pharmaceutical composition, or the food composition in the aspect described above.

In some embodiments, the method for improving sleep described above excludes medical practices on humans (e.g., a method of treating humans).

In some embodiments, the method for improving sleep comprises administering to a non-human animal any one of the peptides (I) to (IV) described above. The sleep improvement may be the prevention or treatment of a sleep disorder. The method for improving sleep may be a method for preventing or treating a sleep disorder, comprising administering any one of the peptides (I) to (IV) to a non-human animal. Any one of the peptides (I) to (IV) may be administered to a non-human animal in an amount effective for preventing or treating a sleep disorder.

In some embodiments, the method for improving sleep comprises administering any one of the peptides (I) to (IV) described above to a human. The sleep improvement may exclude medical practices on humans (e.g., a method of treating humans). Alternatively, the sleep improvement may be the prevention or treatment of a sleep disorder in humans. The method for improving sleep may be a method for preventing or treating a sleep disorder, comprising administering to a human any one of the peptides (I) to (IV) described above. Any one of the peptides (I) to (IV) described above can be administered to a human in an amount effective for preventing or treating a sleep disorder.

### Industrial Applicability

Mouse orexin-C has action of suppressing wakefulness and inducing sleep in mice, and therefore orexin-C may be effective in treating insomnia. Additionally, although therapeutic methods for hypersomnia (narcolepsy) have been difficult to develop, they may be achieved by suppressing the action of orexin-C. Thus, orexin-C is a novel target for improving sleep-wake abnormalities.

The disclosures of all patent applications and documents cited in the present specification are incorporated herein by reference in their entirety.

The following Examples are intended for illustration only and are not intended to limit the technical scope of the present invention in any way. Unless otherwise specified, the reagents are commercially available, or obtained or prepared according to commonly used techniques in the art or procedures described in known literature.

### Examples

### 1. Experimental Method

C57BL/6J mice (male, 8 weeks old) were anesthetized by inhalation with isoflurane, equipped with electrodes for electroencephalogram analysis (Pinnacle Technology, KS, USA) and a guide cannula for intracerebroventricular administration, and then allowed to recover for one week. After habituation for 3 days to an electroencephalogram-electromyogram data acquisition device for sleep-wake analysis (Pinnacle Technology), mouse orexin-C (mOXC, 3 nmol, SEQ ID NO: 2) or phosphate-buffered saline (PBS) as a control was administered intracerebroventricularly into the lateral ventricle of the mice (0.3 mm posterior and 0.9 mm lateral to the bregma, and 2.2 mm from the brain surface), and electroencephalograms and electromyograms were recorded for 12 hours. The polypeptide of mOXC was prepared by synthesis. Further, after at least 3 days had elapsed, PBS or mOXC (3 nmol) was administered to each group of mice as a crossover study, and electroencephalograms and electromyograms were recorded for 12 hours. The results of these two consecutive experiments were combined and evaluated as a two-group comparison test between the mOXC-administered group and the PBS-administered group. Waking time, REM sleep time, and non-REM sleep time were calculated using SleepSign software (Kissei Comtec, Nagano, Japan). A statistical significance was detected using Student's paired *t*-test.

### 2. Results

As shown in Figs. 1(A) to 1(F), when mOXC (3 nmol) was intracerebroventricularly administered to mice at ZT12 (the start time of the waking period of mice), waking time decreased (Figs. 1(A) and 1(D)), REM sleep time increased slightly (Figs. 1(B) and 1(E)), and non-REM sleep time increased significantly (Figs. 1(C) and 1(F)).

Sequence Listing:

## Claims

1. An agent for sleep improvement, comprising any one of the following peptides (I) to (IV):
(I) a peptide containing the amino acid sequence of the C-terminal peptide of preproorexin,
(II) a peptide having at least 90% amino acid sequence identity to the amino acid sequence of the C-terminal peptide of preproorexin of the peptide (I),
(III) a peptide that is a part of the amino acid sequence of the C-terminal peptide of preproorexin of the peptide (I), and
(IV) a variant of the peptide (I) or (III) in which one or two amino acids are deleted, substituted, added, or inserted with respect to the peptide (I) or (III).

2. The agent for sleep improvement according to claim 1, wherein the peptide (I) is a peptide containing the amino acid sequence of any one of SEQ ID NOs: 1 to 3.

3. The agent for sleep improvement according to claim 1, which is a sleep inducer.

4. The agent for sleep improvement according to claim 1, wherein the sleep improvement includes an improvement in sleep depth or an improvement in asleep onset.

5. The agent for sleep improvement according to claim 1, which is an agent for the prevention or treatment of a sleep disorder.

6. The agent for sleep improvement according to any one of claims 1 to 5, which is in the form of a pharmaceutical composition.

7. The agent for sleep improvement according to any one of claims 1 to 5, which is in the form of a food composition.

8. The agent for sleep improvement according to any one of claims 1 to 5, which is in the form of feed.

9. A method for improving sleep, comprising administering to a subject any one of the following peptides (I) to (IV), excluding a method for treating a human:
(I) a peptide containing the amino acid sequence of the C-terminal peptide of preproorexin,
(II) a peptide having at least 90% amino acid sequence identity to the amino acid sequence of the C-terminal peptide of preproorexin of the peptide (I),
(III) a peptide that is a part of the amino acid sequence of the C-terminal peptide of preproorexin of the peptide (I), and
(IV) a variant of the peptide (I) or (III) in which one or two amino acids are deleted, substituted, added, or inserted with respect to the peptide (I) or (III).

10. The method according to claim 9, wherein the sleep improvement includes prevention or treatment of a sleep disorder.
